# EUROPEAN PATENT SPECIFICATION

(11) **EP 1 879 840 B1**
(45) Date of publication and mention of the grant of the patent: **29.12.2010**
(21) Application number: 06751624.5
(22) Date of filing: 25.04.2006
(51) Int. Cl.: C07C 17/154

(54) **OXIDATIVE HALOGENATION OF C1 HYDROCARBONS TO HALOGENATED C1 HYDROCARBONS**
OXIDATIVE HALOGENIERUNG VON C1-KOHLENWASSERSTOFFEN ZU HALOGENIERTEN C1-KOHLENWASSERSTOFFEN
HALOGENATION OXYDATIVE D'HYDROCARBURES C1 EN HYDROCARBURES C1 HALOGENES

(30) Priority: 04.05.2005 US 677591 P
(43) Date of publication of application: 23.01.2008
(73) Proprietor: DOW GLOBAL TECHNOLOGIES INC., Midland MI 48674 (US)
(72) Inventor: PODKOLZIN, Simon, G., Midland, MI 48642 (US); STANGLAND, Eric, E., Midland, MI 48642 (US); SCHWEIZER, Albert, E., Jr., Midland, MI 48642 (US); JONES, Mark, E., Midland, MI 48642 (US)
(74) Representative: Prins, Adrianus Willem
(86) International application number: PCT/US2006/015993
(87) International publication number: WO 2006/118935

(56) References cited:
- EP-A2- 0 156 634
- WO-A-02/094749
- US-B1- 6 452 058

## Description

### Background of the Invention

This invention pertains to a process for the oxidative halogenation of methane and halogenated C₁ hydrocarbons. For the purposes of this discussion, the term "oxidative halogenation" shall refer to a process wherein methane or a halogenated C₁ hydrocarbon (the "C₁ reactant hydrocarbon") is contacted with a source of halogen and a source of oxygen in the presence of a catalyst under process conditions sufficient to form a halogenated C₁ product having at least one additional halogen substituent as compared with the C₁ reactant hydrocarbon. As an example of this process, reference is made to contacting methane with hydrogen chloride and oxygen in the presence of a catalyst to form methyl chloride.

Monohalogenated methanes, such as methyl chloride, find utility in producing silicones and higher halogenated methanes, and as intermediates in the production of numerous commodity chemicals, such as methanol, dimethyl ether, light olefins, gasoline, vinyl chloride, and acetic acid. Higher halogenated C₁ compounds, such as dichloromethane, find utility as solvents, as intermediates in the manufacture of silicones, and in the methylation or etherification of cellulose, alcohols, and phenols.

As supplies and access to crude oil become more uncertain, exploration is proceeding into alternative sources of hydrocarbons and fuel. The conversion of natural gas, containing predominantly low molecular weight alkanes, to higher molecular weight hydrocarbons has received consideration, because large deposits of natural gas, chiefly composed of methane, are found in many locations throughout the world. In addition, low molecular weight alkanes are generally present in coal deposits and can be formed during mining operations, during various petroleum processes, and during the gasification or liquefaction of synthetic fuelstocks, such as, coal, tar sands, oil shale, and biomass. In the search for petroleum, large amounts of natural gas are often discovered in remote parts of the world, where few local markets exist for use as a fuel or chemical feedstock.

Readily accessible natural gas is typically used in local markets as fuel for residential, commercial, and industrial applications. Typically, materials used as fuel are traded at prices below the prices commanded for chemical feedstocks. Use of natural gas as a chemical feedstock is therefore a high-value application. Accessibility, however, is a major obstacle to the effective and extensive use of remote gas, whether for fuel or feedstock. Disadvantageously, vast quantities of natural gas are flared or vented in remote geographical areas from which transport in gaseous form is effectively impossible.

Conversion of natural gas to useful chemical feedstocks, preferably liquid feedstocks, offers a promising solution to the problem of transporting low molecular weight hydrocarbons from remote locations; but conversions of this sort present a special challenge to the petrochemical and energy industries. The dominant technology now employed for utilizing remote natural gas involves its conversion to synthesis gas (syngas), a mixture of hydrogen and carbon monoxide, with the syngas subsequently being converted to liquid products. Synthesis gas, for example, can be converted to syncrude by Fischer-Tropsch technology; and syncrude can be upgraded to transportation fuels using typical refining methods. Alternatively, synthesis gas can be converted to liquid oxygenates, such as methanol, which in turn can be converted to more conventional transportation fuels via zeolitic catalysts.

While syngas processing provides a means for converting natural gas into a more easily transportable liquid that in turn can be converted into useful chemical products, the intermediate step involved in such processing, i.e., the formation of the synthesis gas, is costly. Accordingly, a search continues for alternate, less costly, means of converting methane directly into more valuable chemical feedstocks.

A potential alternate route to activating methane involves its oxidative halogenation in a first step to form methyl halide or other lower halogenated methanes, e.g., dihalomethanes, which in turn can be converted into valuable commodity chemicals, such as methanol, dimethyl ether, light olefins, higher hydrocarbons, including gasoline, as well as vinyl chloride, and acetic acid. When applied to chlorine halogenation, this route has been referred to as the "chlorine-assisted" route, which can be represented by the following two-step process (1) and (2):

CH₄ + HCl + ½ O₂ → chloromethane(s) + H₂O (1)

chloromethane(s) → chemical product + HCl (2)

For such a reaction scheme to be practical, hydrogen chloride generated in the second step should be recycled to the first step of the process.

Typically, prior art oxidative halogenation processes, for example, US 4,769,504, and US 4,795,843, produce a large quantity of perhalogenated product, such as carbon tetrachloride, which depending upon the end use may offer less value than lower halogenated products, such as methyl chloride and dichloromethane. As a further disadvantage, prior art processes produce an unacceptable quantity of deep oxidation products (COₓ), specifically, carbon monoxide and carbon dioxide. The production of oxidized products irretrievably wastes the C₁ hydrocarbon feed and creates product separation and by-product disposal problems. As a further disadvantage, prior art processes produce elemental chlorine, which decreases the target utilization of the chlorine source, may be corrosive to the process equipment, and may require additional separation steps. As a further disadvantage, many transition metal halides used as catalysts in these processes exhibit significant vapor pressure at reaction temperatures. This volatility generally produces a decline in catalyst activity and/or deposition of corrosive materials in downstream parts of the process equipment.

Other art, exemplified by US-B1-6,452,058 and WO 02/94749, discloses the oxidative halogenation of C₁ hydrocarbons with a source of chlorine and, optionally, a source of oxygen in the presence of a rare earth halide or rare earth oxyhalide catalyst, in the substantial absence of copper or iron, to yield methyl chloride. The molar ratio of C₁ reactant hydrocarbon to source of halogen is disclosed to range from greater than about 1/1 to less than about 20/1. The molar ratio of C₁ reactant hydrocarbon to source of oxygen is disclosed to range from greater than about 2/1 to less than about 20/1, with from about 2/1 to about 10/1 being preferred. Advantageously, the disclosed process tends to produce more of the valuable mono- and di-halogenated products and less of the higher halogenated products. The process, however, results in a large quantity of unconverted hydrogen chloride in the effluent stream. Hydrogen chloride and by-product water form an azeotrope from which it is difficult and expensive to separate dry hydrogen chloride for recycle to the process. Moreover, handling a mixture of reactant hydrocarbon and oxygen presents safety issues. Finally, the catalyst should be desirably operated at elevated temperatures to Improve process productivity; but higher temperatures result in lower selectivity to desired halogenated C₁ products. Thus, while US-B1-6,452,058 offers advantages as compared with other prior art processes, improvements are desirable, if the technology is to be commercialized.

### Summary of the invention.

This invention provides for a novel and improved oxidative halogenation process fro preparing a halogenated C₁ product. The invention comprises contacting in a reactor a C₁ reactant hydrocarbon selected from the group consisting of methane, halogenated C₁ hydrocarbons, and mixtures thereof with a source of oxygen and a source of halogen in the presence of a catalyst under process conditions sufficient to prepare a halogenated C₁ product having at least one additional halogen substituent as compared with the C₁ reactant hydrocarbon. In one important aspect, the process of this invention is conducted with a feed to the reactor having a molar ratio of reactant hydrocarbon to source of halogen greater than 23/1; or with a feed to the reactor having a molar ratio of reactant hydrocarbon to source of oxygen greater than about 46/1. Alternatively, the process may be operated under conditions wherein the molar ratio of reactant hydrocarbon to source of oxygen is greater than 23/1 and the molar ratio of reactant hydrocarbon to source of oxygen is greater than about 46/1. The catalyst used in the process of this invention comprises a rare earth oxyhalide or rare earth halide substantially free of iron and copper, such that the atom ratio of rare earth element to iron and copper is greater than 10/1, with the proviso that when cerium is present in the catalyst, then at least one other rare earth element is also present in the catalyst.

The oxidative halogenation process of this invention advantageously converts a C₁ reactant hydrocarbon selected from methane and halogenated C₁ hydrocarbons to a halogenated C₁ product having at least one additional halogen substituent as compared with the reactant hydrocarbon. The process of this invention operates at high molar ratio of C₁ reactant hydrocarbon to source of halogen and/or at high molar ratio of C₁ reactant hydrocarbon to source of oxygen. Under these conditions, the conversion of C₁ reactant hydrocarbon is effectively limited, thereby resulting in improved selectivity to halogenated C₁ product, preferably monohalogenated C₁ product. A selectivity of greater than about 90 mole percent C₁ halogenated product is typically achieved. More advantageously, a low selectivity to undesirable oxygenates, such as, carbon monoxide and carbon dioxide, is achieved. The lower selectivity to oxygenated by-products correlates with a more efficient use of reactant hydrocarbon, a higher productivity of the desired halogenated C₁ product, and fewer separation and waste disposal problems. The selectivity advantage obtained from this process invention allows for operation at higher process temperatures, which beneficially results in higher catalyst productivity.

In addition, the catalyst employed in the process of this invention does not require a conventional carrier or support, such as alumina or silica. Rather, the catalyst employed in this invention beneficially comprises a rare earth halide or rare earth oxyhalide that uniquely functions both as a catalyst support and as a source of a catalytically active rare earth component. Unlike many heterogeneous catalysts of the prior art, the rare earth halide catalyst of this invention is beneficially soluble in water. Accordingly, should process equipment, such as filters, valves, circulating tubes, and small or intricate parts of reactors, become plugged with particles of the rare earth halide catalyst, advantageously, a simple water wash can dissolve the plugged particles and restore the equipment to working order. As a further advantage, the rare earth halide and rare earth oxyhalide catalysts employed in the process of this invention exhibit acceptable reaction rates and evidence of long lifetimes. Essentially no deactivation of these catalysts has been observed over the run times tested.

In preferred embodiments of this invention, the process can be advantageously engineered to increase process productivity and decrease, or even eliminate effluent separation and recycle problems. Specifically, the process may be run to essentially complete conversion of the source of halogen, thereby avoiding the cost and effort required to separate a dry stream of unconverted source of halogen from the product stream for recycle to the process. Such separation efforts are typically complicated by the presence of by-product water in the product stream. Water and the unconverted source of halogen, e.g., hydrogen chloride, form an azeotropic mixture that is highly corrosive to the process equipment and difficult to separate. By operating at essentially complete conversion of source of halogen, the preferred process of this invention avoids the aforementioned problems.

In another preferred embodiment, the process of this invention may be run over a pre-halogenated catalyst, in the absence of a flow of the halogen source (i.e., methane and oxygen only with a feed to the reactor having a molar ratio of C₁ reactant hydrocarbon to source of halogen equal to essentially infinity; or alternatively, a molar ratio of source of halogen to C₁ reactant hydrocarbon equal to essentially zero. With respect to the aforementioned ratios, the words "essentially infinity" and "essentially zero" will apply when the source of halogen is not fed to the reactor with the C₁ reactant hydrocarbon and oxygen, and the concentration of source of halogen in the feed is less than about 0.5 volume percent, preferably, less than about 0.1 volume percent.). This method of operation advantageously increases the selectivity of halogenated C₁ product to essentially 100 mole percent while also advantageously eliminating the requirement for the separation of the unconverted source of halogen from the product stream for recycle to the process. Process operation without a flow of halogen source can be sustained by periodically halogenating the catalyst by employing, without limitation, a pulse mode, a swing mode, or a circulating bed reactor, as explained in detail hereinafter. In this mode of operation, the catalyst functions both as catalyst and source of halogen.

In another preferred embodiment, the process of this invention may be engineered to operate at essentially complete conversion of the source of oxygen; thereby increasing the selectivity of halogenated C₁ product to essentially 100 percent, while reducing safety problems associated with handling mixtures of hydrocarbons and oxygen and eliminating downstream separation of oxygen from the hydrocarbons. Finally, in other preferred embodiments of this invention, the process can be run at elevated temperatures beneficially to increase catalyst productivity with little or no sacrifice of selectivity to desired halogenated C₁ product.

All of the aforementioned properties render the process of this invention uniquely attractive for converting methane and halogenated C₁ hydrocarbons into more highly halogenated C₁ hydrocarbons, including methyl chloride, for multiple uses in downstream industrial chemical processes.

### Detailed Description of the Invention

In the oxidative halogenation process of this invention, a halogenated C₁ product, preferably a monohalogenated C₁ hydrocarbon product, is produced, more preferably, in a selectivity greater than about 90 mole percent. In even more preferred embodiments, the process achieves essentially 100 mole percent selectivity to halogenated C₁ product. Advantageously low levels of undesirable COₓ oxygenates (CO and CO₂) are obtained.

This novel and improved oxidative halogenation process comprises contacting in a reactor a C₁ reactant hydrocarbon selected from the group consisting of methane, C₁ halogenated hydrocarbons, and mixtures thereof with a source of halogen and a source of oxygen in the presence of a catalyst under process conditions sufficient to prepare a halogenated C₁ product having at least one additional halogen substituent as compared with the C₁ reactant hydrocarbon. In an important aspect of this invention, the feed to the reactor is maintained at a molar ration of C₁ reactant hydrocarbon to source of halogen greater than 23/1. Alternatively, the feed to the reactor is maintained at a molar ratio of C₁ reactant hydrocarbon to source of oxygen greater than about 46/1. As a further alternative, the feed to the reactor may be maintained such that both the molar ratio of the C₁ reactant hydrocarbon to the source of halogen is at a value greater than 23/1 and the molar ratio of the C₁ reactant hydrocarbon to the source of oxygen is at a value greater than about 46/1. The unique catalyst employed in the oxidative halogenation process of this invention comprises a rare earth halide or rare earth oxyhalide compound that is substantially free of iron and copper, such that the atom ratio of rare earth element to iron and copper is greater than 10/1, with the further proviso that when cerium is present in the catalyst, then at least one other rare earth element is also present in the catalyst.

In a preferred embodiment, the process is conducted to essentially complete conversion of the source of halogen, thereby eliminating or reducing separation and safety problems associated with mixtures of hydrocarbons and oxygen. The words "essentially complete conversion of the source of halogen" are intended to mean a conversion of source of halogen greater than about 95 mole percent.

When both the source of halogen and source of oxygen are run to essentially complete conversion, then a product stream comprising unconverted C₁ reactant hydrocarbon, halogenated C₁ product, water, and residual quantities, if any, of the source of halogen and the source of oxygen is obtained as the effluent from the reactor. From this product stream, halogenated C₁ product and water are separated out, resulting in a recycle stream comprising unconverted C₁ reactant hydrocarbon and residual, if any, quantities of source of halogen and source of oxygen. The recycle stream is typically fed directly to the reactor in step (a) without further processing. The catalyst can act as a buffer or sorbent to remove residual source of halogen from the recycle stream. Generally, residual oxygen, if any, is well below the explosive and flammability limits, and is not typically problematical; although the aforementioned statement does not relieve the skilled artisan from taking adequate precautions.

In another preferred aspect, this invention provides for improved engineering by separating catalyst halogenation from the production of the halogenated hydrocarbon product. Such engineering is accomplished by operating in pulse or swing mode, said process comprising:
(a) introducing into a reactor containing a catalyst a flow of a feed comprising a source of halogen, the catalyst comprising a rare earth halide or rare earth oxyhalide, the rare earth halide or oxyhalide being substantially free of iron and copper, such that the atom ratio of rare earth element to iron and copper is greater than 10/1, with the proviso that when cerium is present in the catalyst, then at least one other rare earth element is also present in the catalyst;
(b) stopping the flow of the source of halogen to the reactor;
(c) introducing into the reactor a flow of a feed comprising a C₁ reactant hydrocarbon selected from the group consisting of methane, C₁ halogenated hydrocarbons, and mixtures thereof and a source of oxygen, such that a concentration of source of halogen in said flow is less than 0.5 volume percent and molar ratio of C₁ reactant hydrocarbon to source of halogen is greater than 23/1. under process conditions sufficient to prepare a halogenated C₁ product having at least one additional halogen substituent as compared with the reactant hydrocarbon;
(d) stopping the flow of the feed comprising the C₁ reactant hydrocarbon and the source of oxygen; and
(e) repeating steps (a) through (d) in an alternating fashion.
In pulse mode operation, the aforementioned process is typically conducted in one reactor with alternating flows, first of halogen, and then of a mixture of C₁ reactant hydrocarbon and oxygen. In swing mode, the catalyst is reacted with a halogen source in one reactor, while a feed comprising the C₁ reactant and a source of oxygen is reacted over a prehalogenated catalyst in a second reactor; and then the reactant feeds to the two reactors are interchanged, or swung. This separation of catalyst halogenation from the production of a halogenated hydrocarbon can also be accomplished in a circulating bed reactor, wherein a fraction of the catalyst bed is continuously withdrawn from the hydrocarbon reactor, sent to a regenerator and reacted with a halogen source. Subsequently, the halogenated catalyst is returned to the hydrocarbon reactor.

It is to be understood that in the pulse, swing or circulating bed mode, the pre-halogenated catalyst provides the source of halogen. Under such circumstances, it is to be understood that the concentration of source of halogen in the feed to the reactor of C₁ reactant hydrocarbon and oxygen is less than 0.5 volume percent, preferably, less than about 0.1 volume percent, and more preferably, approaches essentially zero (within detectable limits), because the source of halogen is not fed. Thus, the molar ratio of the C₁ reactant hydrocarbon to source of halogen in the feed to the reactor is a value much in excess of 23/1 and effectively approaches infinity or, in other words, the molar ratio of source of halogen to C₁ reactant hydrocarbon in the feed effectively approaches zero.

In an even more preferred embodiment, the process of this invention provides for the oxidative halogenation of methane to a halogenated methane, preferably, in a selectivity greater than about 95 mole percent. In this even more preferred embodiment, the process comprises contacting methane with a source of halogen and a source of oxygen, at a molar ratio of methane to source of halogen in the feed to the reactor of greater than 23/1 and/or at a molar ratio of methane to source of oxygen in the feed to the reactor of greater than about 46/1; under reaction conditions and in the presence of the aforementioned rare earth halide or rare earth oxyhalide catalyst.

In a most preferred embodiment, the process of this invention provides for the oxidative monochlorination of methane to form methyl chloride in accordance with the stoichiometric reaction shown in Equation (3):

CH₄ + HCl + ½ O₂ → CH₃Cl + H₂O (3)

Preferably, the molar ratio of methane to oxygen is chosen for operation outside the fuel-rich explosive and flammability limits of the mixture. More preferably, the process comprises contacting methane with oxygen and hydrogen chloride at a molar ratio of methane to hydrogen chloride in the feed to the reactor greater than about 30/1 and/or at a molar ratio of methane to oxygen in the feed to the reactor greater than about 60/1; in the presence of a catalyst comprising lanthanum chloride or lanthanum oxychloride, substantially absent copper and iron as described hereinabove, and under reaction conditions sufficient to form methyl chloride.

The novel oxidative halogenation process of this invention may be beneficially integrated with downstream processes to convert methyl halides into highly valuable commodity chemicals, including methyl alcohol, dimethyl ether, light olefins, such as ethylene, propylene, and butenes; and higher hydrocarbons, including C5+ gasolines; as well as vinyl halide monomer and acetic acid. Details of these downstream processes are set forth hereinafter.

The C₁ reactant hydrocarbon used in the oxidative halogenation process of this invention comprises methane, a halogenated C₁ hydrocarbon, or a mixture thereof, capable of acquiring halogen substituents in accordance with the process described herein. The halogen substituent of the halogenated C₁ hydrocarbon is preferably selected from the group consisting of chlorine, bromine, and iodine, more preferably, chlorine and bromine. One, two, or three halogen substituents may be present on the halogenated C₁ hydrocarbon; but for the purposes of this invention, the C₁ reactant has at least one carbon-hydrogen bond and does not comprise a perhalogenated compound, such as carbon tetrachloride. Preferably, when a halogenated C₁ hydrocarbon is employed, only one or two halogen substituents are present, as exemplified by methyl chloride or dichloromethane. Different halogen substituents may be suitably present in the C₁ hydrocarbon reactant, as exemplified by bromochloromethane.

Suitable examples of halogenated C₁ hydrocarbons include, without limitation, methyl chloride, methyl bromide, methyl iodide, dichloromethane, dibromomethane, diiodomethane, chloroform, tribromomethane, bromodichloromethane, iododichloromethane, chlorodibromomethane, iododibromomethane, and the like. Methane, however, is the most preferred C₁ reactant hydrocarbon. The C₁ reactant hydrocarbon may be provided to the oxidative halogenation process as a pure feed stream, or diluted with an inert diluent as described hereinafter, or as a mixture of methane and halogenated C₁ hydrocarbon, optionally, further in combination with an inert diluent.

The source of halogen, which is employed in the process of this invention, may be any inorganic or organic halogen-containing compound (or mixture of such compounds) that is capable of transferring its halogen atom(s) to the reactant hydrocarbon. Suitable non-limiting examples of the source of halogen include chlorine, bromine, iodine, hydrogen chloride, hydrogen bromide, hydrogen iodide, and halogenated hydrocarbons having one or more labile halogen substituents (i.e., transferable halogen substituents), the latter preferably being perhalocarbons or highly halogenated hydrocarbons having two or more halogen atoms. Non-limiting examples of perhalocarbons with labile halogen substituents include carbon tetrachloride and carbon tetrabromide. Non-limiting examples of highly halogenated hydrocarbons having two or more halogen substituents, at least one substituent of which is labile, include chloroform and tribromomethane. Preferably, the source of halogen is a source of chlorine or a source of bromine, more preferably, hydrogen chloride or hydrogen bromide, most preferably, hydrogen chloride. In a further aspect of this invention, the catalyst may be pre-halogenated to form a halogenated catalyst. In this embodiment of the invention, the catalyst may function dually as the catalyst and the source of halogen.

The source of halogen may be provided to the process in any amount that is effective in producing the desired halogenated C₁ product. Typically, the amount of halogen source in the feed will vary depending upon the specific process stoichiometry, the reactor design, and safety considerations. Broadly, the molar ratio of C₁ reactant hydrocarbon to source of halogen in the feed may range from about 1/1 to essentially infinity (∞). For the purposes of this invention, the term "infinity" will correlate with a concentration of source of halogen in the feed to the reactor of less than about 0.5 volume percent, and preferably, less than about 0.1 volume percent. Typically for such oxidative halogenation processes, the molar ratio of C₁ reactant hydrocarbon to source of halogen in the feed to the reactor is greater than about 1/1, preferably, greater than about 4/1; but for the purposes of this invention, the molar ratio of C₁ reactant hydrocarbon to source of halogen is greater than about 23/1, even more preferably, greater than about 40/1. The upper limit on the molar ratio of C₁ reactant hydrocarbon to source of halogen in the feed depends on the mode of operation. In the presence of a flow of source of halogen, typically, the molar ratio of C₁ reactant hydrocarbon to source of halogen in the feed is less than about 70/1, preferably, less than about 60/1, and more preferably, less than about 50/1. In pulse, swing or circulating bed mode, the source of halogen is not fed with the C₁ reactant hydrocarbon and oxygen; thus the molar ratio of C₁ reactant hydrocarbon to source of halogen in the feed is effectively infinity or, in other words, the molar ratio of source of halogen to C₁ reactant hydrocarbon in the feed is essentially zero, with the catalyst itself providing the source of halogen, as noted hereinbefore.

The source of oxygen can be any oxygen-containing gas or mixture of such gases, such as, essentially pure molecular oxygen, air, oxygen-enriched air, or a mixture of oxygen with a diluent gas that does not interfere with the oxidative halogenation process, such as, nitrogen, argon, helium, carbon monoxide, carbon dioxide, methane, and mixtures thereof. Typically for such oxidative halogenation processes, the molar ratio of C₁ reactant hydrocarbon to source of oxygen in the feed to the reactor is greater than about 1/1, preferably, greater than about 4/1; but for the purposes of this invention, the molar ratio of C₁ reactant hydrocarbon to source of oxygen is greater than about 46/1, even more preferably, greater than about 50/1, and most preferably, greater than about 60/1. In continuous flow reactors, the molar ratio of reactant hydrocarbon to oxygen in the feed is typically less than about 140/1, preferably, less than about 120/1, and more preferably, less than about 100/1.

In the process of this invention, it is required to maintain the molar ratio of reactant C₁ hydrocarbon to source of halogen at a value greater than 23/1; or alternatively, to maintain the molar ratio of reactant C₁ hydrocarbon to source of oxygen at a value greater than about 46/1; or alternatively, to maintain both ratios at the high values so specified. Operating at either of said molar ratios effectively limits the conversion of C₁ reactant hydrocarbon. In turn, the selectivity to desired halogenated C₁ product is significantly and unexpectedly enhanced. Maintaining the molar ratio of reactant C₁ hydrocarbon to source of halogen at a value greater than 23/1 is equivalent to maintaining the molar ratio of reactant C₁ hydrocarbon to source of oxygen at a value greater than 46/1 for the purpose of limiting the maximum conversion of reactant C₁ hydrocarbon, based on the stoichiometry shown in equations (1) and (3) hereinabove.

Optionally, if desired, the feed, comprising reactant hydrocarbon, source of halogen, and source of oxygen, can be diluted with a diluent or carrier gas constituting any essentially non-reactive gas, that is, a gas that does not substantially interfere with the oxidative halogenation process. The diluent may assist in removing products and heat from the reactor and in reducing the number of undesirable side-reactions. Non-limiting examples of suitable diluents include nitrogen, argon, helium, carbon monoxide, carbon dioxide, and mixtures thereof. In an alternative embodiment, methane may be used as a diluent, although methane is reactive in this process. The quantity of diluent employed is typically greater than about 10 mole percent, and preferably, greater than about 20 mole percent, based on the total moles of feed to the reactor, including total moles of reactant hydrocarbon, source of halogen, source of oxygen, and diluent. The quantity of diluent employed is typically less than about 90 mole percent, and preferably, less than about 70 mole percent, based on the total moles of feed to the reactor.

The catalyst employed in the oxidative halogenation process of this invention generically comprises a rare earth compound, substantially absent of iron and copper, as noted hereinafter. Moreover, when cerium is present in the catalyst, then at least one additional rare earth material is desirably present in the catalyst. The rare earths are a group of 17 elements consisting of scandium (atomic number 21), yttrium (atomic number 39) and the lanthanides (atomic numbers 57-71) [James B. Hedrick, U.S. Geological Survey - Minerals Information - 1997, "Rare-Earth Metals"]. Preferably, herein, the term is taken to mean an element selected from lanthanum, cerium, neodymium, praseodymium, dysprosium, samarium, yttrium, gadolinium, erbium, ytterbium, holmium, terbium, europium, thulium, lutetium, and mixtures thereof. Preferred rare earth elements for use in the aforementioned oxidative halogenation process are those that are typically considered as being single valency metals. The catalytic performance of rare earth compounds with multi-valency metals appears to be less desirable than rare earth compounds with single valency metals, as explained hereinafter. The rare earth element for this invention is preferably selected from the group consisting of lanthanum, neodymium, praseodymium, dysprosium, yttrium, and mixtures thereof. Most preferably, the rare earth element used in the catalyst is lanthanum or a mixture of lanthanum with other rare earth elements.

In a preferred aspect, the rare earth oxyhalide or rare earth halide may be represented by the following stoichiometric formula:

MO_{y}X_{z}

wherein "M" represents a rare earth element or mixture of rare earth elements; "O" is oxygen; "y" is any number ranging from 0 to 1.5; "X" is a halide; and "z" is any number ranging from greater than 0 to 3.0.

In one preferred form, the rare earth halide is represented by the formula MX₃, wherein M is at least one rare earth element selected from the group consisting of lanthanum, cerium, neodymium, praseodymium, dysprosium, samarium, yttrium, gadolinium, erbium, ytterbium, holmium, terbium, europium, thulium, lutetium, and mixtures thereof; and wherein X is selected from the group consisting of chloride, bromide, iodide, and mixtures thereof. More preferably, X is chloride, and the more preferred rare earth halide is represented by the formula MCl₃, wherein M is defmed hereinbefore. Most preferably, X is chloride, and M is lanthanum or a mixture of lanthanum with other rare earth elements.

In another preferred embodiment, the rare earth halide is porous, meaning that typically the rare earth halide has a BET surface area of greater than about 3 m²/g, preferably, greater than about 5 m²/g. More preferably, the BET surface area is greater than about 10 m²/g, even more preferably, greater than about 15 m²/g, as an even higher preference, greater than about 20 m²/g, and most preferably, greater than about 30 m²/g. Generally, the BET surface area of the rare earth halide is less than about 200 m²/g. For these above measurements, a nitrogen adsorption isotherm was measured at 77K and the surface area was calculated from the isotherm data utilizing the BET method (S. Brunauer, P. H. Emmett, and E. Teller, Journal of the American Chemical Society, 60, 309 (1938)).

In yet another preferred embodiment, the catalyst employed in this invention comprises a rare earth oxyhalide, represented by the formula MOX, wherein M is at least one rare earth element selected from the group consisting of lanthanum, cerium, neodymium, praseodymium, dysprosium, samarium, yttrium, gadolinium, erbium, ytterbium, holmium, terbium, europium, thulium, lutetium, and mixtures thereof; and wherein X is selected from the group consisting of chloride, bromide, iodide, and mixtures thereof. More preferably, the rare earth halide is a rare earth oxychloride, represented by the formula MOCl, wherein M is defined hereinbefore. Most preferably, M is lanthanum or lanthanum with a mixture of other rare earth elements.

In a preferred embodiment, the rare earth oxyhalide is also porous, which for the oxyhalide generally implies a BET surface area of greater than about 12 m²/g. Preferably, the rare earth oxyhalide has a BET surface area of greater than about 15 m²/g, more preferably, greater than about 20 m²/g, and most preferably, greater than about 30 m²/g. Generally, the BET surface area of the rare earth oxyhalide is less than about 200 m²/g. In addition, it is noted that the MOCl phases possess characteristic powder X-Ray Diffraction (XRD) patterns that are distinct from the MCl₃ phases.

Although the catalyst may be loaded into the reactor in a preferred MOX or MX₃ form, under reaction conditions the stoichiometry of the catalyst may vary throughout the process in accordance with the variations implied by formula [MO_{y}X_{z}], identified hereinabove.

In general, the presence in the catalyst of metals that are capable of oxidation-reduction (redox) is undesirable. Redox metals typically include transition metals that have more than one stable oxidation state, such as iron, copper, and manganese. The rare earth halide or oxyhalide catalyst of this invention is specifically required to be substantially free of copper and iron. The term "substantially free" means that the atom ratio of rare earth element to redox metal, preferably iron or copper, is greater than about 10/1, more preferably greater than about 15/1, and most preferably greater than about 50/1. In addition, cerium, a lanthanide rare earth element, is known to be an oxidation-reduction catalyst having the ability to access both the +3 and +4 oxidation states. For this reason, if the rare earth metal is cerium, the catalyst of this invention further comprises at least one more rare earth metal other than cerium. Preferably, if one of the rare earth metals is cerium, the cerium is provided in a molar ratio that is less than the total amount of other rare earth metals present in the catalyst. More preferably, however, substantially no cerium is present in the catalyst. By "substantially no cerium" it is meant that any cerium present is in an amount less than about 10 atom percent, preferably, less than about 5 atom percent, and even more preferably, less than about 1 atom percent of the total rare earth components.

In an alternative embodiment of this invention, the rare earth halide or rare earth oxyhalide catalyst, described hereinbefore, may be bound to, extruded with, or deposited onto a catalyst support, such as alumina, silica, silica-alumina, porous aluminosilicate (zeolite), silica-magnesia, bauxite, magnesia, silicon carbide, titanium oxide, zirconium oxide, zirconium silicate, or any combination thereof. In this embodiment, the total concentration of active rare earth metals on the support is typically greater than about 0.01 weight percent and typically less than about 50 weight percent, based on the total weight of the catalyst, including the support.

It may also be advantageous to include other elements within the catalyst. For example, preferable elemental additives include alkali and alkaline earths, boron, phosphorous, sulfur, germanium, titanium, zirconium, hafnium, and combinations thereof. These elements can be present to alter the catalytic performance of the composition or to improve the mechanical properties (e.g. attrition-resistance) of the material. In a preferred embodiment, the elemental additive is calcium. The total concentration of elemental additives in the catalyst is typically greater than about 0.01 weight percent and typically less than about 20 weight percent, based on the total weight of the catalyst.

The rare earth halide and rare earth oxyhalide compounds may be obtained commercially or prepared by methods published in the art. A method currently felt to be preferable for forming the porous rare earth oxyhalide (MOX) comprises the following steps: (a) preparing a solution of a halide salt of the rare earth element or elements in a solvent comprising either water, an alcohol, or mixtures thereof; (b) adding a base to cause the formation of a precipitate; and (c) collecting and calcining the precipitate in order to form the MOX. Preferably, the halide salt is a rare earth chloride salt, for example, any commercially available rare earth chloride. Typically, the base is a nitrogen-containing base selected from ammonium hydroxide, alkyl amines, aryl amines, arylalkyl amines, alkyl ammonium hydroxides, aryl ammonium hydroxides, arylalkyl ammonium hydroxides, and mixtures thereof. The nitrogen-containing base may also be provided as a mixture of a nitrogen-containing base with other bases that do not contain nitrogen. Preferably, the nitrogen-containing base is ammonium hydroxide or tetra(alkyl)ammonium hydroxide, more preferably, tetra(C₁₋₂₀ alkyl)ammonium hydroxide. Porous rare earth oxychlorides may also be produced by appropriate use of alkali or alkaline earth hydroxides, particularly, with the buffering of a nitrogen-containing base, although caution should be exercised to avoid producing substantially the rare earth hydroxide or oxide. The solvent in Step (a) is preferably water. Generally, the precipitation is conducted at a temperature greater than about 0°C. Generally, the precipitation is conducted at a temperature less than about 200°C, preferably, less than about 100°C. The precipitation is conducted generally at about ambient atmospheric pressure, although higher pressures may be used, as necessary, to maintain liquid phase at the precipitation temperature employed. The calcination is typically conducted at a temperature greater than about 200°C, preferably, greater than about 300°C, and less than about 800°C, preferably, less than about 600°C. Production of mixed carboxylic acid and rare earth chloride salts also can yield rare earth oxychlorides upon appropriate decomposition.

A method currently felt to be preferable for forming the porous rare earth halide (MX₃) catalyst comprises the following steps: (a) preparing a solution of a halide salt of the rare earth element or elements in a solvent comprising either water, an alcohol, or mixtures thereof; (b) adding a base to cause the formation of a precipitate; (c) collecting, washing and calcining the precipitate; and (d) contacting the calcined precipitate with a halogen source. Preferably, the rare earth halide is a rare earth chloride salt, such as any commercially available rare earth chloride. The solvent and base may be any of those mentioned hereinbefore in connection with the formation of MOX. Preferably, the solvent is water, and the base is a nitrogen-containing base, as previously described. The precipitation is generally conducted at a temperature greater than about 0°C and less than about 200°C, preferably less than about 100°C, at about ambient atmospheric pressure or a higher pressure so as to maintain liquid phase. The calcination is typically conducted at a temperature greater than about 200°C, preferably, greater than about 300°C, but less than about 800°C, and preferably, less than about 600°C. Preferably, the halogen source is a hydrogen halide, such as hydrogen chloride, hydrogen bromide, or hydrogen iodide. More preferably, the halogen source is hydrogen chloride. The contacting with the halogen source is typically conducted at a temperature greater than about 100°C and less than about 500°C. Typical pressures for the contacting with the source of halogen range from about ambient atmospheric pressure to pressures less than about 150 psia (1,034 kPa).

As noted hereinabove, the rare earth oxyhalide (MOX) compound can be converted into the rare earth halide (MX₃) compound by treating the oxyhalide with a source of halogen. Since the process of this invention requires a source of halogen, it is possible to contact the rare earth oxyhalide with a source of halogen, such as chlorine, in the oxidative halogenation reactor to form the MX₃ catalyst *in situ.* Alternatively, the rare earth oxyhalide can be halided in a pretreatment step prior to initiating the oxidative halogenation process; and then the process itself can be conducted in a continuous or intermittent flow of halogen.

The oxidative halogenation process of this invention can be conducted in a reactor of any conventional design suitable for the gas phase reaction chemistry, including batch, fixed bed, fluidized bed, transport bed, continuous and intermittent flow reactors, catalytic distillation reactors, and pulse mode and swing mode reactors. The process conditions (for example, molar ratio of feed components, temperature, pressure, gas hourly space velocity), can be varied provided that the desired halogenated C₁ product, preferably monohalogenated C₁ hydrocarbon product, more preferably, methyl chloride, is obtained. At the high molar ratios of C₁ reactant hydrocarbon to source of oxygen used in this process invention, elevated process temperatures may be employed for increased catalyst productivity. Beneficially, the higher process temperatures can be employed without sacrifice to product selectivity.

Generally, therefore, the process temperature is greater than about 375°C, preferably, greater than about 400°C, and more preferably, greater than about 475°C. Generally, the process temperature is less than about 700°C, preferably, less than about 650°C, and more preferably, less than about 600°C. Ordinarily, the process can be conducted at atmospheric pressure; but operation at higher or lower pressures is possible, as desired. Preferably, the pressure is equal to or greater than about 14 psia (97 kPa), but less than about 150 psia (1,034 kPa). Typically, the total weight hourly space velocity (WHSV) of the total feed (reactant hydrocarbon, source of halogen, source of oxygen, and optional diluent) is greater than about 0.1 gram total feed per g catalyst per hour (h⁻¹), and preferably, greater than about 0.5 h⁻¹. Typically, the total weight hourly space velocity of the total feed is less than about 100 h⁻¹, and preferably, less than about 20 h⁻¹.

Desirably, the reactor for the process is designed to maximize catalyst productivity; maximize selectivity to halogenated C₁ product; minimize quantities of unconverted sources of halogen and oxygen; maximize safety; and minimize separation and recycle efforts. Preferred reactor designs include pulse mode and swing mode. In pulse mode, a flow of a source of halogen is implemented first to halogenate the catalyst. Then, the flow of the source of halogen is stopped; and a flow comprising the C₁ hydrocarbon reactant and source of oxygen is contacted with the catalyst to produce the halogenated C₁ product. When the catalyst is essentially deactivated by dehalogenation, the flow of hydrocarbon reactant and source of oxygen is stopped. Starting again with the halogenation of the catalyst under a flow of source of halogen, the process steps are repeated in alternating pulses through the duration of operation.

In a variant of the pulse mode, the process may be operated in swing mode using multiple reactor beds. Typically, two or more reactor beds containing the catalyst are employed. In swing mode, a flow of the source of halogen is contacted with a first catalyst bed. When the first catalyst bed is activated, the flow of source of halogen is stopped, and the flow is swung over to halogenate another catalyst bed. While the other bed is being activated, a flow of a feed comprising a C₁ reactant hydrocarbon and source of oxygen is contacted with the activated first catalyst bed to produce a halogenated C₁ product. As the first catalyst bed becomes deactivated and the second catalyst bed becomes fully activated, the flows are switched, or swung, such that the source of halogen is again contacted with the first catalyst bed, while the C₁ hydrocarbon reactant and source of oxygen are contacted with the second bed. The swing mode allows for continuous operation and production of the product without the interruption or downtime characteristic of the pulse mode. Pulse and swing reactors are known in the art.

Another suitable reactor involves a reactor with a circulating catalyst bed, wherein a portion of the catalyst bed is continuously circulated between a first reactor for hydrocarbon oxyhalogenation and a second reactor for catalyst regeneration. Thus, a mixture comprising the C₁ hydrocarbon reactant and source of oxygen are contacted with an activated catalyst in halogenated form in the first reactor to form halogenated C1 product; while a portion of the catalyst is continuously regenerated under the source of halogen in the catalyst regeneration reactor.

In any embodiment of the invention described hereinabove, it is preferable to drive the conversion of the source of halogen essentially to completion, thereby minimizing the amount of unconverted source of halogen in the product stream. In this manner costly efforts are avoided to recover a dry stream of unconverted source of halogen from the product stream for recycle to the reactor. It is also preferable to drive the conversion of the source of oxygen essentially to completion. This latter option offers increased safety of handling and avoids downstream oxygen separation, because the quantity of unconverted oxygen in the product stream, if any at all, should fall below the fuel-rich explosive and flammability limits of a mixture of hydrocarbon and oxygen. In a more preferred manner of operation, conversions of both the source of halogen and the source of oxygen are driven essentially to completion.

By operating in the above-described manner, a product stream comprising unconverted C₁ hydrocarbon reactant, halogenated C₁ product(s), water, and residual quantities, if any, of source of halogen and source of oxygen is obtained. The product stream may be processed by conventional means, such as distillation and extraction, to recover the halogenated C₁ product(s) and to separate water formed as a by-product of the process. After such processing, a recycle stream comprising unconverted C₁ hydrocarbon reactant, and residual source of halogen and source of oxygen, if any, is recycled directly to the oxidative halogenation reactor without further treatment. Any residual source of halogen that may be present in the hydrocarbon recycle stream can be absorbed by the oxidative halogenation catalyst, which acts as a buffer or sorbent to remove residual halogen.

When the oxidative halogenation process is conducted as described hereinabove, then a halogenated C₁ product is formed that has at least one additional halogen substituent as compared with the reactant hydrocarbon. Halogenated C₁ products beneficially produced by the oxidative halogenation process of this invention include, without limitation, methyl chloride, dichloromethane, methyl bromide, dibromomethane, methyl iodide, chloroform, tribromomethane, and to some extent starting from trihalogenated reactants, carbon tetrachloride and carbon tetrabromide. Preferably, the halogenated C₁ product is a monohalogenated C₁ hydrocarbon. More preferably, the halogenated C₁ hydrocarbon product is methyl chloride or methyl bromide; most preferably, methyl chloride.

For the purposes of the description herein, "conversion" shall be defined as the mole percentage of reactant compound that is converted, i.e., reacted, in the oxidative halogenation process of this invention to form product(s). Reference may be made to "conversion of reactant hydrocarbon," or "conversion of source of halogen," or "conversion of source of oxygen." Conversions vary depending upon the specific reactant being considered, specific catalyst, and specific process conditions. Typically, for the process of this invention, the conversion of methane or reactant halogenated C₁ hydrocarbon is greater than about 0.5 mole percent, preferably, greater than about 1.0 mole percent, and more preferably, greater than 2.0 mole percent. Typically, for the process of this invention, the conversion of the source of halogen is greater than about 20 mole percent, preferably, greater than about 50 mole percent, more preferably, greater than about 70 mole percent, and most preferably, greater than about 95 mole percent. Typically, the conversion of source of oxygen is greater than about 20 mole percent, preferably, greater than about 50 mole percent, more preferably, greater than about 70 mole percent, and most preferably, greater than about 95 mole percent.

For the purposes of this invention, "selectivity" shall be defined as the mole percentage of converted methane or reactant halogenated C₁ hydrocarbon that is converted into a specific product, for example, a halogenated C₁ product or an oxygenated by-product, such as CO or CO₂. In the oxidative halogenation process of this invention, the selectivity to halogenated C₁ product having one additional halogen substituent as compared with the C₁ hydrocarbon reactant (most preferably, the product being methyl chloride or methyl bromide starting from methane) is typically greater than about 87 mole percent, preferably, greater than about 90 mole percent, more preferably, greater than about 92 mole percent, and most preferably, greater than about 98 mole percent. For the most preferred methane reactant, the selectivity to dihalogenated C₁ hydrocarbon product, preferably dichloromethane or dibromomethane, is typically less than about 10 mole percent, and preferably, less than about 5 mole percent; and essentially no trihalogenated or perhalogenated product is found. By "essentially no trihalogenated or perhalogenated product," it is meant that not more than about 5 mole percent total of said species is produced from methane, preferably, not more than about 2 mole percent, and most preferably not more than about 1 mole percent produced from methane, based on the moles of converted methane.

As a further advantage, in preferred embodiments of this invention low levels of oxygenated by-products, such as COₓ oxygenates (CO and CO₂) are produced. Typically, the total selectivity to carbon monoxide and carbon dioxide is less than about 10 mole percent, preferably, less than about 5 mole percent, and more preferably, less than about 3 mole percent, based on the moles of converted methane or converted C₁ reactant hydrocarbon. Surprisingly, the use in pulse or swing mode of a stream comprising reactant hydrocarbon and oxygen does not increase undesirable and wasteful oxygenated by-products (COx); and unexpectedly the desirable selectivity to halogenated C₁ product(s) is maintained. Considering the high operating temperatures of the process, the result is even more unexpected.

The monohalogenated and dihalogenated C₁ products produced in the oxidative halogenation process of this invention can be utilized as feeds in downstream processes for manufacture of high-value commodity chemicals, such as, methyl alcohol, dimethyl ether, light olefins, including ethylene, propylene, and butenes; higher hydrocarbons, including C5+ gasolines; vinyl halide monomer, and acetic acid. Conditions for processing methyl chloride into such commodity chemicals are known in the art and described briefly hereinafter.

The hydrolysis of methyl halides to form methyl alcohol is disclosed, for example, in US 1,086,381, US 4,990,696, US 4,523,040, US 5,969,195, and disclosed by G. Olah in Journal of the American Chemical Society, 1985, 107, 7097-7105, and I. Fells, Fuel Society Journal, 10, 1959, 26-35.

For the example of methyl chloride hydrolysis to methyl alcohol, the process can be represented by the following stoichiometric reaction (4):

CH₃Cl + H₂O → CH₃OH + HCl (4)

Many catalysts exhibit activity for this hydrolysis process including, for example, alumina; various zeolites of the ZSM structure code, such as ZSM-5, preferably, having a Constraint Index from 1 to 12; alkali and alkaline earth metal hydroxides and alkoxides, such as sodium hydroxide, potassium hydroxide, and sodium ethoxide; alkyl ammonium hydroxides and various amines, for example, trimethylamine hydroxide and piperidine; transition metal halide complexes, preferably, halide complexes of platinum, palladium, and nickel, and mixtures thereof, more preferably, the chloride complexes thereof, optionally including a cation of H⁺, Group IA, or Group IIA elements, such as K⁺ or Na⁺; and metal oxide/hydroxide catalysts, including the metal oxides/hydroxides of Group IIA elements (e.g., Mg, Ba); as well as the entire series of transition elements (e.g., V, Cr, Zr, Ti, Fe, or Zn), supported on γ-alumina or activated carbon.

The hydrolysis process conditions can vary depending upon the particular catalyst and alkyl halide employed. Since the thermodynamics favor the reverse reaction to form methyl halide (i.e., Equation 4 in reverse), an excess of water relative to methyl halide is typically employed to drive the equilibrium towards methyl alcohol. Preferably, the molar ratio of water to methyl halide is greater than about 1:1, more preferably, greater than about 5:1. Preferably, the water/methyl halide molar ratio is less than about 20:1, more preferably, less than about 10:1. Generally, the hydrolysis is conducted at a temperature greater than about 85°C, and preferably, greater than about 115°C. Generally, the hydrolysis is conducted at a temperature less than about 600°C, and preferably, less than about 400°C. The process pressure can also vary from subatmospheric to superatmospheric; but generally ranges from greater than about 7 psia (50 kPa), and preferably, greater than about 14 psia (97 kPa), to less than about 725 psia (4,999 kPa), and preferably, less than about 73 psia (500 kPa). The weight hourly space velocity (WHSV) of the methyl halide feed can vary widely from a value typically greater than about 0.1 g feed per g catalyst per hour (h⁻¹) to a value less than about 1,000 h⁻¹. Preferably, the weight hourly space velocity of the methyl halide feed ranges from greater than about 1 h⁻¹ to less than about 10 h⁻¹.

The conversion of methyl halide, that is, the mole percentage of methyl halide converted relative to methyl halide in the feed, will vary depending upon the specific catalyst and process conditions. Generally, methyl alcohol and dimethyl ether are the predominant products in varying ratios depending upon the catalyst and process conditions. Further details of the hydrolysis process and product distribution can be found in the pertinent references cited hereinabove. Hydrogen halide, which is a co-product of the hydrolysis process, can be conveniently recycled to the oxidative halogenation reactor, where it is consumed as a source of halogen.

In another aspect of this invention, the methyl halide prepared by the aforementioned oxidative halogenation of methane can be condensed to form light olefins, such as ethylene, propylene, butenes, and higher hydrocarbons, including C5+ gasolines. For the example of methyl chloride being converted into ethylene, the stoichiometric reaction can be represented by the following Equation (5):

2 CH₃Cl → CH₂=CH₂ + 2 HCl (5)

As seen from the above, hydrogen halide, such as hydrogen chloride, is produced as a co-product of this condensation process. Again, the hydrogen halide can be conveniently recycled to the oxidative halogenation reactor and consumed as a source of halogen.

Any catalyst capable of effecting the condensation process can be employed. US 5,397,560, for example, discloses the use of aluminosilicates having a DCM-2 structure code for the conversion of methyl halides into light olefins, predominantly ethylene and propylene. Catalysts known for the condensation of methyl alcohol to light olefins and gasolines can also be employed analogously for the condensation described herein of methyl halides into light olefins and gasolines. Non-limiting examples of such catalysts include zeolites of the ZSM structure code, such as ZSM-5, ZSM-11, ZSM-12, ZSM-34, ZSM-35, and ZSM-38, preferably, wherein the aforementioned ZSM zeolite has a Constraint Index from 1 to 12; as well as various aluminophosphates (ALPO's) and silicoaluminophosphates (SAPO's). References disclosing one or more of the aforementioned catalysts include US 3,894,107, US 4,480,145, US 4,471,150, US 4,769,504, US 5,912,393.

Generally, the condensation process involves contacting methyl halide with the catalyst under condensation process conditions sufficient to prepare at least one light olefin, such as ethylene, propylene, butenes, or at least one C5+ hydrocarbon, or any mixture thereof. The process temperature typically is greater than about 250°C, and preferably, greater than about 350°C. The process temperature is typically less than about 600°C, and preferably, less than about 450°C. The process pressure can vary from subatmospheric to superatmospheric; but generally a pressure greater than about 0.1 psi absolute (689 Pa) and less than about 300 psi absolute (2,068 kPa) is employed. The weight hourly space velocity (WHSV) of the methyl halide feed can vary widely from a value typically greater than about 0.1 g feed per g catalyst per hour (h⁻¹) to a value less than about 1,000 h⁻¹. Preferably, the weight hourly space velocity of the methyl halide feed ranges from greater than about 1 h⁻¹ to less than about 10 h⁻¹. The product distribution of the aforementioned condensation process will vary depending upon the specific feed, catalyst, and process conditions. A product stream comprising light olefins, predominantly ethylene, propylene, and butenes, is usually obtained with the DCM-2 catalyst. A product stream containing predominantly heavier hydrocarbons, such as C5+ gasolines, is usually obtained with zeolite ZSM catalysts.

In a further application of this invention, ethylene obtained from the condensation of methyl halide can be fed directly into a vinyl halide monomer process, wherein the ethylene is contacted with a source of halogen, preferably hydrogen halide, and optionally, a source of oxygen in the presence of an oxidative halogenation catalyst. Preferably, a source of oxygen is used. For the purposes of making vinyl halide monomer, the source of halogen and the source of oxygen can be any of those sources of halogen and sources of oxygen described hereinbefore in connection with the oxidative halogenation of methane. For the purposes of preparing vinyl halide monomer, the oxidative halogenation catalyst can be any conventional catalyst known for such a purpose, including supported copper catalysts, such as, supported copper chloride promoted with alkali or alkaline earth halides, known to those skilled in the art. When these conventional catalysts are used, then dihaloethane is obtained, which is subsequently thermally cracked to vinyl halide monomer. In a preferred embodiment, the oxidative halogenation catalyst is the rare earth halide or rare earth oxyhalide catalyst described hereinbefore in connection with the oxidative halogenation process of this invention. When the rare earth halide or oxyhalide is used, then vinyl halide is obtained directly without the need for a separate thermal cracking reactor. Vinyl halide can also be made by mixing ethylene with the methane feed to the methane oxidative halogenation reactor so as to obtain an effluent containing both methyl halide and vinyl halide. Separation of methyl halide and vinyl halide prior to conversion of the methyl halide to ethylene beneficially provides a two-reactor system for producing vinyl halide from methane.

Typically, in the preparation of vinyl halide the molar ratio of ethylene to oxygen is greater than about 2/1, preferably, greater than about 4/1, and generally, less than about 20/1, and preferably, less than about 15/1. Generally, the oxidative halogenation of ethylene is carried out at a temperature greater than about 150°C, preferably, greater than about 200°C, and more preferably, greater than about 250°C. Typically, the oxidative halogenation of ethylene is carried out at a temperature less than about 500°C, preferably, less than about 425°C, and more preferably, less than about 350°C. Ordinarily, the process will be conducted at atmospheric pressure or a higher pressure. Typically, then, the pressure will be equal to or greater than about 14 psia (101 kPa), but less than about 150 psia (1,034 kPa). Typically, the total gas hourly space velocity (GHSV) of the reactant feed (ethylene, source of halogen, source of oxygen, and any optional diluent) will vary from greater than about 10 ml total feed per ml catalyst per hour (h⁻¹), preferably, greater than about 100 h⁻¹, to less than about 50,000 h⁻¹, and preferably, less than about 10,000 h⁻¹. Further details on catalyst and process conditions suitable for the oxidative halogenation of ethylene-containing streams to vinyl halide monomer can be found in WO 01/38273, WO 01/38274, and WO 01/42176.

In yet another aspect of this invention, the methyl halide, produced in the oxidative halogenation of methane, can be carbonylated with a carbonylation agent in the presence of a carbonylation catalyst to form acetyl halide, which thereafter can be hydrolyzed to form acetic acid. Any carbonylation process conditions can be used, provided that the carbonylation yields the desired acetyl halide product. The carbonylation agent, itself, can be any compound that is capable of transferring carbonyl (CO) to the methyl halide. Preferably, the carbonylation agent is carbon monoxide or an organometallic complex containing labile carbon monoxide, such as, transition metal salts and complexes, including Group VIII salts and complexes, such as the salts and complexes of palladium, iron, and cobalt, further including the carbonyl complexes of said transition metals. The molar ratio of carbonylation agent to methyl halide is typically at least 1:1, and preferably, greater than 1:1. More preferably, the molar ratio of carbonylation agent to methyl halide is greater than about 2:1. Preferably, the molar ratio of carbonylation agent to methyl halide is less than about 20:1, more preferably, less than about 10:1. Generally, the carbonylation step is conducted at a temperature greater than about 50°C and at a temperature less than about 350°C. The pressure may range typically from atmospheric to higher pressures, generally from greater than about 7 psia (50 kPa) to less than about 725 psia (4,999 kPa). The total weight hourly space velocity (WHSV) of the carbonylation feed, including methyl halide and carbonylation agent, can vary widely from a value typically greater than about 0.1 g feed per g catalyst per hour (h⁻¹) to a value less than about 1,000 h⁻¹.

The product of the carbonylation process is acetyl halide, preferably, acetyl chloride. The subsequent hydrolysis of acetyl halide to acetic acid is readily effected by contacting acetyl halide with water under process conditions sufficient to form acetic acid. One skilled in the art will know the details of the hydrolysis of acetyl halide, as this step is a straight-forward hydrolysis of an acyl halide described, for example, in numerous organic chemistry textbooks.

### Example 1

A catalyst composition comprising a porous lanthanum oxychloride was prepared as follows. Lanthanum chloride (LaCl₃ 7 H₂O, 60 g) was dissolved in deionized water (500 ml) in a round-bottom flask. The solution was sparged with argon for 1 hour. Ammonium hydroxide (6 M, 80 ml) was added to the solution with stirring. A white precipitate was formed, and the resulting slurry was stirred under argon for 1 hour. The mixture was centrifuged (3100 rpm, 15 min), and the excess liquid was decanted to yield a solid. The solid was dried at 70°C for 12 hours; then calcined in an air flow by ramping the temperature to 450°C in 1 hour, holding at 450°C for 1 hour, then ramping to 550°C over 1 hour, and then holding at 550°C for 1 hour. The calcined solid was characterized as LaOCl, based on X-ray diffraction and elemental analysis.

The catalyst prepared hereinabove was crushed to 20 x 40 US mesh (0.85 x 0.43 mm) and evaluated in the oxidative chlorination of methane as follows. A tubular, nickel-alloy reactor, having a ratio of length to diameter of 28.6/1 {6 inches (15.24 cm) x 0.210 inches (0.533 cm)} was loaded with catalyst (3.16 g). Prior to reaction the catalyst was treated with a flow of hydrogen chloride (5 mole percent) in helium at a total flow of 30 standard cubic centimeters per minute (sccm) for 12 hours. The reactor was then fed a mixture of methane, hydrogen chloride, and oxygen in the ratios shown in Table 1. Operating pressure was atmospheric at the reactor outlet. Operating temperature and gas hourly space velocity are as shown in Table 1. Exit gases were analyzed by gas phase chromatography with the results set forth in Table 1.

In this and subsequent experiments, conversion was measured as the relative difference in inlet and outlet molar concentrations of specified reactant: (Cᵢₙ - Cₒᵤₜ)/Cᵢₙ x 100. Selectivity to a specific C₁ product was measured as the molar ratio of that product in the outlet stream to the sum of all products in the outlet stream: Cₒᵤₜ/ΣCₒᵤₜ x 100. The detection limit for various feed and product components was 0.01 mole percent. No other carbon-containing products other than those listed in the tables were found in any of the experiments. Catalyst productivity was calculated as the amount of methyl chloride (in kg) produced per kg of catalyst per hour.

**Table 1. Methane Oxidative Chlorination to Methyl Chloride at Elevated CH₄/HCl and CH₄/O₂ Mole Ratios**

| Exp. | CH₄/ HCl Ratio | CH₄/ O₂ Ratio | CH₄/HCl/O₂ mol % | T °C | WHSV h⁻¹ | Conv. CH₄ mol % | Conv. HCl mol % | Conv. O₂ mol % | Sel. CH₃Cl mol % | Sel. CH₂Cl₂ mol % | Sel. CHCl₃ mol% | Sel. CO mol % | Sel. CO₂ mol % | Productivity CH₃Cl kg h⁻¹ (kg cat)⁻¹ |
|---|---|---|---|---|---|---|---|---|---|---|---|---|---|---|
| 1a | 31.8 | 63.7 | 95.5/3.0/1.5 | 450 | 0.71 | 0.6 | 20.4 | 20.4 | 100.0 | 0.0 | 0.0 | 0.0 | 0.0 | 0.01 |
| lb | 31.8 | 63.7 | 95.5/3.0/1.5 | 475 | 0.71 | 1.0 | 32.1 | 32.1 | 99.2 | 0.8 | 0.0 | 0.0 | 0.0 | 0.02 |
| 1c | 31.8 | 63.7 | 95.5/3.0/1.5 | 540 | 0.71 | 2.2 | 66.7 | 76.5 | 90.5 | 2.5 | 0.0 | 0.0 | 7.0 | 0.04 |
| 1d | 23.5 | 47.0 | 94.0/4.0/2.0 | 527 | 7.38 | 0.7 | 16.5 | 16.5 | 100.0 | 0.0 | 0.0 | 0.0 | 0.0 | 0.14 |
| le | 23.5 | 47.0 | 94.0/4.0/2.0 | 527 | 1.43 | 2.4 | 57.5 | 58.7 | 96.0 | 3.0 | 0.0 | 0.0 | 1.0 | 0.09 |
| 1f | 23.5 | 47.0 | 94.0/4.0/2.0 | 527 | 0.69 | 3.2 | 76.8 | 79.6 | 94.1 | 4.0 | 0.0 | 0.0 | 1.9 | 0.06 |
| CE-1a | 5.0 | 20.0 | 80.0/16.0/4.0 | 500 | 0.75 | 5.5 | 29.7 | 60.5 | 90.0 | 9.0 | 0.0 | 0.0 | 1.0 | 0.08 |
| CE-1b | 5.0 | 20.0 | 80.0/16.0/4.0 | 525 | 0.74 | 7.5 | 41.3 | 84.0 | 88.0 | 11.0 | 0.0 | 0.0 | 1.0 | 0.10 |

From Table 1 it is seen that a selectivity to methyl chloride of greater than 90 mole, and upwards of 100 percent within detectable limits, can be achieved at elevated molar ratios of methane to oxygen and high operating temperatures.

### Comparative Experiment CE-1

For comparative purposes, methane was oxidatively halogenated in the presence of hydrogen chloride and oxygen in the presence of the catalyst and in accordance to the procedure of Example 1, with the exception that the methane/hydrogen chloride molar ratio was only 5.0/1 rather than 23.1/1 or 31.8/1, and the methane/oxygen molar ratio was only 20.0/1 rather than 47.0/1 or 63.7/1. Results are shown in Table 1. It is seen from Table 1, CE-1a and CE-1b, that as temperature increased, the selectivity to monohalogenated product decreased. Thus, it became more difficult to operate at higher temperatures and maintain selectivity. However, when experiment CE-1a is compared with Example 1c, it is seen that comparable selectivity to methyl chloride was achieved at higher temperature, provided that the molar ratio of methane/hydrogen chloride was greater than 23/1 and the molar ratio of methane/oxygen was greater than 46/1. Moreover, when experiment CE-1b is compared with Examples 1d-f, it is further substantiated that considerably higher selectivity to methyl chloride was achieved at a temperature greater than 500°C, provided that the molar ratio of methane/hydrogen chloride was greater than 23/1, and the molar ratio of methane to oxygen was greater than 46/1. Moreover, under these process conditions, the selectivity to methyl chloride was as high as 100 percent, within detectable limits (expt. 1(d)). Operation at higher temperatures offered increased catalyst productivity, as noted in Table 1.

### Example 2

The process of Example 1 was repeated, with the exception that the mole ratio of methane/hydrogen chloride was set at a value higher than 45/1 and the mole ratio of methane/oxygen was set at a value higher than 60/1. Hydrogen chloride was reacted to essentially 100 percent conversion. In Example 2c, both hydrogen chloride and oxygen were reacted to essentially 100 percent conversion. Process conditions and results are shown in Table 2.

**Table 2. Methane Oxidative Chlorination to Methyl Chloride at High Conversion of HCl and Oxygen at Elevated CH₄/HCl and CH₄/O₂ Mole Ratios**

| Exp 2 | CH₄/ HCl Ratio | CH₄ / O₂ Ratio | CH₄/HCl/O₂/N₂ mol % | T °C | WHSV h⁻¹ | Conv CH₄ mol % | Conv HCl mol % | Conv O2 mol % | Sel CH₃Cl mol % | Sel CH₂Cl₂ mol % | Sel CHCl₃ mol % | Sel CO mol % | Sel CO₂ mol % | Prod CH₃Cl kg h⁻¹ (kg cat)⁻¹ |
|---|---|---|---|---|---|---|---|---|---|---|---|---|---|---|
| a | 48.3 | 64.3 | 96.5/2.0/1.5/0.0 | 540 | 0.71 | 2.3 | 100.0 | 80.3 | 88.9 | 2.0 | 0.0 | 0.0 | 9.1 | 0.03 |
| b | 54.1 | 70.8 | 92.0/1.7/1.3/5.0 | 540 | 0.71 | 2.0 | 100.0 | 77.3 | 90.7 | 1.3 | 0.0 | 0.0 | 8.0 | 0.03 |
| c | 54.2 | 76.8 | 92.1/1.7/1.2/5.0 | 540 | 0.71 | 2.2 | 100.0 | 100.0 | 83.6 | 0.2 | 0.0 | 0.0 | 16.2 | 0.04 |

In Examples 2a-c selectivity to methyl chloride remained at an acceptable level even as the conversions of hydrogen chloride and oxygen reached values close to 100 percent within detectable limits. The elimination of hydrogen chloride through complete conversion eliminates the need to recover a dry hydrogen chloride stream from aqueous hydrogen chloride in the product stream. The elimination of oxygen through complete conversion eliminates the need for oxygen separation from the hydrocarbons.

### Example 3

A lanthanum oxychloride material was prepared and loaded into a tubular reactor in a manner similar to that described in Example 1 hereinabove. The lanthanum oxychloride was chlorinated at 400°C under a stream of hydrogen chloride at ambient pressure for a period of 12 hours. The flow of hydrogen chloride was stopped; and a pulse of methane (10.0 mole percent), oxygen (5.0 mole percent), helium (83.0 mole percent), and argon (2.0 mole percent) was injected into the reactor at 450°C. Molar ratio of methane to oxygen in the feed to the reactor was 2/1; while the concentration of source of halogen in the feed to the reactor was essentially zero (less than the detection limit of 0.01 mole percent). The catalyst, halogenated in the first step of the process, provided the halide to the second reaction step of the process. A mass spectroscopy analysis of the effluent as a function of time shows the presence of methane, oxygen, inert gases, and methyl chloride as a primary product. This experiment illustrates the process in a pulse mode, and the feasibility of conducting the process in a swing or circulating reactor bed mode. Surprisingly, the selectivity to methyl chloride was not impaired by using a flow of only methane and oxygen.

### Example 4

The oxidative chlorination of methane was conducted over a lanthanum oxychloride catalyst in a manner similar to that described in Example 1, with the exception of using the process conditions shown in Table 3, expt. 4(a). Notably, the start-up conditions of 4(a) involved a molar ratio of methane to hydrogen chloride in the feed to the reactor of 0.20/1, the ratio being chosen to keep the catalyst in the substantially chlorinated form. The molar ratio of methane to oxygen in the feed to the reactor was 2/1.

**Table 3. Methane Oxidative Chlorination to Methyl Chloride with ((a) and (c)) and without (b) Source of Chlorine in the Reactor Feed.**

| Expt. | CH₄/HCl/O₂/N₂ mol % | T °C | WHSV h⁻¹ | Conv. CH₄ mol % | Sel. CH₃Cl mol % | Sel. CH₂Cl₂ mol % | Sel. CHCl₃ mol % | Sel. CO mol % | Sel. CO₂ mol % | Prod. CH₃Cl kg h⁻¹ (kg cat)⁻¹ |
|---|---|---|---|---|---|---|---|---|---|---|
| 4(a) | 4.8/24.0/2.4/5.0 | 402 | 0.012 | 5.5 | 90 | 10 | 0 | 0 | 0 | 0.0013 |
| 4(b) | 4.8/0.0/2.4/5.0 | 402 | 0.005 | varied | 90-100 | 0-10 | 0 | 0 | 0 | varied |
| 4(c) | 4.8/24.0/2.4/5.0 | 402 | 0.012 | 5.5 | 90 | 10 | 0 | 0 | 0 | 0.0013 |

After 37 minutes on stream, the flow of hydrogen chloride was stopped; and the feed composition was changed to methane and oxygen (with nitrogen diluent to simulate air) at the process conditions specified in Table 3, experiment 4(b). Without hydrogen chloride in the feed (i.e., concentration of hydrogen chloride in the feed to the reactor was essentially zero; molar ratio of methane to hydrogen chloride feed was effectively infinity), the catalyst productivity declined but then stabilized at a lower value, while the selectivity to methyl chloride reached 100 percent. When the hydrogen chloride flow was restored (Table 3, expt. 4(c)), under feed conditions identical to the initial operating conditions (Table 3, expt. 4(a)), the initial catalytic performance, observed prior to the hydrogen chloride elimination, was also restored with regard to both catalyst productivity and selectivity to methyl chloride.

This example illustrates the feasibility of running the process in a pulse, swing or circulating reactor bed mode and at high conversion of source of halogen so as to minimize the amount of unconverted halogen in the effluent stream. Operation at high hydrogen chloride conversion leaves methane and oxygen in the reactor, with surprisingly no significant increase in undesirable oxygenated by-products. Selectivity to methyl chloride remains high. Reduction of hydrogen chloride in the effluent stream advantageously reduces the cost and efforts needed for separation and recovery of hydrogen chloride in the product stream.

## Claims

1. A process of oxidative halogenation comprising contacting in a reactor a C₁ reactant hydrocarbon selected from methane, a halogenated C₁ hydrocarbon, or a mixture thereof with a source of halogen and a source of oxygen in the presence of a catalyst; the molar ratio of C₁ reactant hydrocarbon to source of halogen in a feed to the reactor being greater than 23/1; or the molar ratio of C₁ reactant hydrocarbon to source of oxygen in a feed to the reactor being greater than 46/1; or in both feeds to the reactor the molar ratio of C₁ reactant hydrocarbon to source of halogen being greater than 23/1 and the molar ratio of C₁ reactant hydrocarbon to source of oxygen being greater than 46/1; the contacting being conducted under process conditions sufficient to prepare a halogenated C₁ product having at least one additional halogen substituent as compared with the reactant hydrocarbon; the catalyst comprising a rare earth halide or rare earth oxyhalide substantially free of iron and copper such that the atom ration of rare earth element to iron and copper is greater than 10/1, with the proviso that when cerium is present in the catalyst, then at least one other rare earth element is also present in the catalyst.

2. The process of claim 1 wherein said atom ratio in the catalyst is greater than 15/1, and preferably greater than 50/1.

3. The process of claim 1 wherein the C₁ reactant hydrocarbon is selected from the group consisting of methane, chloromethane, bromomethane, iodomethane, dichloromethane, dibromomethane, diiodomethane, chlorobromomethane, and mixtures thereof.

4. The process of claim 1 wherein the source of halogen is selected from the group consisting of elemental halogens, hydrogen halides, and halogenated hydrocarbons having one or more labile halogen substituents.

5. The process of claim 1 wherein the source of halogen is hydrogen chloride.

6. The process of claim 1 wherein the process is conducted at a molar ratio of C₁ reactant hydrocarbon to source of halogen of greater than 30/1.

7. The process of claim 1 wherein the source of oxygen is selected from the group consisting of molecular oxygen and air; and wherein the process is conducted at a molar ratio of C₁ reactant hydrocarbon to source of oxygen of greater than 50/1.

8. The process of claim 1 wherein the process further comprises a diluent selected from the group consisting of nitrogen, helium, argon, carbon monoxide, carbon dioxide, and mixtures thereof.

9. The process of claim 8 wherein the diluent is used in an amount that is greater than 10 mole percent and less than 90 mole percent, based on the total moles of reactant hydrocarbon and diluent.

10. The process of claim 1 wherein the rare earth halide or rare earth oxyhalide is represented by the formula:
MO_{y}X_{z}
wherein M is one or more rare earth metals; O is oxygen; y is a number ranging from 0 to 1.5; X is a halide; and z is a number ranging from greater than 0 to 3.0.

11. The process of claim 10 wherein M is lanthanum or a mixture of lanthanum with other rare earth elements, and X is chloride.

12. The process of claim 10 wherein the rare earth halide is represented by the formula MX₃ and wherein the rare earth oxyhalide is represented by the formula MOX, wherein M is at least one rare earth selected from the group consisting of lanthanum, cerium, neodymium, praseodymium, dysprosium, samarium, yttrium, gadolinium, erbium, ytterbium, holmium, terbium, europium, thulium, lutetium, and mixtures thereof; and wherein X is chloride, bromide, or iodide.

13. The process of claim 1 wherein the process is conducted at a temperature greater than 375°C and less than 700°C.

14. The process of claim 1 wherein conversion of the source of halogen is greater than 95 mole percent.

15. The process of claim 1 wherein the conversion of the source of oxygen is greater than 95 mole percent.

16. The process of claim 1 wherein the C₁ reactant hydrocarbon is methane; the source of halogen is hydrogen chloride; and the halogenated C₁ product is methyl chloride; and optionally, wherein the methyl chloride is employed in a downstream process to prepare methanol, a light olefin, a gasoline, vinyl chloride monomer, or acetic acid.

17. A process for the production of a halogenated C₁ product comprising:
(a) introducing into a reactor containing a catalyst a flow of a source of halogen, the catalyst comprising a rare earth halide or rare earth oxyhalide, the rare earth halide or oxyhalide being substantially free of iron and copper such that the atom ratio of rare earth element to iron and copper is greater than 10/1, with the proviso that when cerium is present in the catalyst, then at least one other rare earth element is also present in the catalyst;
(b) stopping the flow of the source of halogen to the reactor;
(c) introducing into the reactor a flow of a mixture comprising a C₁ reactant hydrocarbon selected from the group consisting of methane, a halogenated C₁ hydrocarbon, or a mixture thereof, and a source of oxygen, such that a concentration of source of halogen in said flow is less than 0.5 volume percent and the molar ratio of C₁ reactant hydrocarbon to source of halogen is greater than 23/1, under process conditions sufficient to prepare a halogenated C₁ product having at least one additional halogen substituent as compared with the reactant hydrocarbon;
(d) stopping the flow of the mixture comprising the C₁ reactant hydrocarbon and the source of oxygen to the reactor; and
(e) repeating steps (a) through (d) in an alternating fashion.

18. The process of claim 17 wherein said atom ratio in the catalyst is greater than 15/1, and preferably greater than 50/1.

19. The process of claim 17 wherein the process is conducted in pulse mode in one reactor bed; or alternatively, wherein the process is conducted in swing mode using multiple reactor beds, or alternatively using a reactor with a circulating catalyst bed.

20. The process of claim 17 wherein the source of halogen is reacted to a conversion greater than 95 mole percent.

21. The process of claim 17 wherein the source of oxygen is reacted to a conversion greater than 95 mole percent.

22. The process of claim 17 wherein the source of halogen and the source of oxygen are both reacted to a conversion greater than 95 mole percent.

23. The process of claim 17 wherein a product stream comprising unconverted C₁ reactant hydrocarbon, halogenated C₁ product, water, and residual quantities, if any, of source of halogen and source of oxygen is obtained as the effluent from the reactor; and from this product stream, halogenated C₁ product and water are separated resulting in a recycle stream comprising unconverted C₁ reactant hydrocarbon and residual quantities, if any, of source of halogen and source of oxygen; and recycling the recycle stream directly to the reactor to step (c).

24. The process of claim 17 wherein the C₁ reactant hydrocarbon is methane; the source of halogen is hydrogen chloride; and the halogenated C₁ hydrocarbon is methyl chloride; and optionally, wherein the methyl chloride is employed in a downstream process to prepare methanol, a light olefin, a gasoline, vinyl chloride monomer, or acetic acid.

25. The process of claim 17 wherein the temperature is greater than 375°C and less than 700°C.

26. The process of claim 22 wherein the C₁ reactant hydrocarbon is methane; the source of halogen is hydrogen chloride; and the halogenated C₁ hydrocarbon is methyl chloride.

## Patentansprüche

1. Verfahren zur oxidativen Halogenierung, umfassend Inkontaktbringen eines C₁-Kohlenwasserstoff-Reaktanden ausgewählt aus Methan, einem halogenierten C₁-Kohlenwasserstoff oder einer Mischung davon mit einer Halogenquelle und einer Sauerstoffquelle in der Gegenwart von einem Katalysator in einem Reaktor; wobei das molare Verhältnis von C₁-Kohlenwasserstoff-Reaktand zu Halogenquelle in einem Strom zum Reaktor größer als 23/1 ist; oder wobei das molare Verhältnis von C₁-Kohlenwasserstoff-Reaktand zu Sauerstoffquelle in einem Strom zum Reaktor größer als 46/1 ist; oder wobei in beiden Strömen zum Reaktor das molare Verhältnis von C₁-Kohlenwasserstoff-Reaktand zu Halogenquelle größer als 23/1 ist und das molare Verhältnis von C₁-Kohlenwasserstoff-Reaktand zu Sauerstoffquelle größer als 46/1 ist; wobei das Inkontaktbringen unter Verfahrensbedingungen durchgeführt wird, die geeignet sind, ein halogeniertes C₁-Produkt mit mindestens einem zusätzlichen Halogensubstituenten verglichen mit dem Kohlenwasserstoff-Reaktanden herzustellen; wobei der Katalysator, umfassend ein Seltenerdhalogenid oder Seltenerdoxihalogenid, im wesentlichen frei von Eisen und Kupfer ist, so dass das Atomverhältnis von Seltenerdelement zu Eisen und Kupfer größer als 10/1 ist, unter der Bedingung, dass, wenn Cer im Katalysator zugegen ist, dann auch mindestens ein anderes Seltenerdelement in dem Katalysator zugegen ist.

2. Verfahren nach Anspruch 1, wobei das Atomverhältnis in dem Katalysator größer als 15/1 und bevorzugt größer als 50/1 ist.

3. Verfahren nach Anspruch 1, wobei der C₁-Kohlenwasserstoff-Reaktand ausgewählt ist aus der Gruppe bestehend aus Methan, Chlormethan, Brommethan, Iodmethan, Dichlormethan, Dibrommethan, Diiodmethan, Chlorbrommethan und Mischungen davon.

4. Verfahren nach Anspruch 1, wobei die Halogenquelle ausgewählt ist aus der Gruppe bestehend aus elementaren Halogenen, Halogenwasserstoffe und halogenierten Kohlenwasserstoffen mit einem oder mehreren labilen Halogensubstituenten.

5. Verfahren nach Anspruch 1, wobei die Halogenquelle Chlorwasserstoff ist.

6. Verfahren nach Anspruch 1, wobei das Verfahren bei einem molaren Verhältnis von C₁-Kohlenwasserstoff-Reaktand zu Halogenquelle von größer als 30/1 durchgeführt wird.

7. Verfahren nach Anspruch 1, wobei die Sauerstoffquelle ausgewählt ist aus der Gruppe bestehend aus molekularem Sauerstoff und Luft; und wobei das Verfahren bei einem molaren Verhältnis von C₁-Kohlenwasserstoff-Reaktand zu Sauerstoffquelle von größer als 50/1 durchgeführt wird.

8. Verfahren nach Anspruch 1, wobei das Verfahren ferner ein Verdünnungsmittel ausgewählt aus der Gruppe bestehend aus Stickstoff, Helium, Argon, Kohlenmonoxid, Kohlendioxid und Mischungen davon, umfasst.

9. Verfahren nach Anspruch 8, wobei das Verdünnungsmittel in einer Menge, die größer als 10 Molprozent und kleiner als 90 Molprozent, bezogen auf die Gesamtzahl an Molen von Kohlenwasserstoff-Reaktand und Verdünnungsmittel, verwendet wird.

10. Verfahren nach Anspruch 1 , wobei das Seltenerdhalogenid oder Seltenerdoxihalogenid dargestellt wird durch die Formel:
MO_{y}X_{z}
wobei M ein oder mehrere Seltenerdmetall(e) ist/sind; O Sauerstoff ist; y eine Zahl zwischen 0 und 1,5 ist; X ein Halogenid ist; und z eine Zahl ist, die von größer als 0 bis 3,0 variiert.

11. Verfahren nach Anspruch 10, wobei M Lanthan oder eine Mischung aus Lanthan mit anderen Seltenerdelementen ist und X Chlorid ist:

12. Verfahren nach Anspruch 10, wobei das Seltenerdhalogenid dargestellt wird durch die Formel MX₃ und wobei das Seltenerdoxihalogenid dargestellt wird durch die Formel MOX, wobei M mindestens eine seltene Erde ausgewählt ist aus der Gruppe bestehend aus Lanthan, Cer, Neodym, Praseodym, Dysprosium, Samarium, Yttrium, Gadolinium, Erbium, Ytterbium, Holmium, Terbium, Europium, Thulium,
Lutetium und Mischungen davon, und wobei X Chlorid, Bromid oder Iodid ist.

13. Verfahren nach Anspruch 1, wobei das Verfahren bei einer Temperatur größer als 375 °C und kleiner als 700°C durchgeführt wird.

14. Verfahren nach Anspruch 1, wobei die Umsetzung der Halogenquelle größer als 95 Molprozent ist.

15. Verfahren nach Anspruch 1, wobei die Umsetzung der Sauerstoffquelle größer als 95 Molprozent ist.

16. Verfahren nach Anspruch 1, wobei der C₁-Kohlenwasserstoff-Reaktand Methan ist; die Halogenquelle Chlorwasserstoff ist; und das halogenierte C₁-Produkt Methylchlorid ist und wobei gegebenenfalls das Methylchlorid in einem nachgeschalteten Verfahren, um Methanol, ein leichtes Olefin, Benzin, Vinylchloridmonomer oder Essigsäure herzustellen, eingesetzt wird.

17. Verfahren zur Herstellung eines halogenierten C₁-Produkts, umfassend:
(a) Einbringen eines Stroms einer Halogenquelle in einen Reaktor, wobei der Reaktor einen Katalysator enthält und wobei der Katalysator ein Seltenerdhalogenid oder Seltenerdoxihalogenid umfasst, wobei das Seltenerdhalogenid oder -oxihalogenid im wesentlichen frei von Eisen und Kupfer ist, so dass dass Atomverhältnis von Seltenerdelement zu Eisen und Kupfer größer ist als 10/1 unter der Voraussetzung, dass, wenn Cer in dem Katalysator zugegen ist, dann auch mindestens ein anderes Seltenerdelement in dem Katalysator zugegen ist;
(b) Beenden des Stroms der Halogenquelle zum Reaktor;
(c) Einführen eines Flusses einer Mischung, umfassend einen C₁-Kohlenwasserstoff-Reaktanden ausgewählt aus der Gruppe bestehend aus Methan, einem halogenierten C₁-Kohlenwasserstoff oder einer Mischung davon, und einer Sauerstoffquelle in den Reaktor, so dass eine Konzentration der Halogenquelle in dem Fluss weniger als 0,5 Volumenprozent ist und das molare Verhältnis von C₁-Kohlenwasserstoff-Reaktand zu Halogenquelle größer als 23/1 ist, unter Verfahrensbedingungen, die geeignet sind, um ein halogeniertes C₁-Produkt mit mindestens einem zusätzlichen Halogensubstituenten verglichen mit dem Kohlenwasserstoff-Reaktanden, herzustellen;
(d) Beenden des Flusses der Mischung, umfassend den C₁-Kohlenwasserstoff-Reaktanden und die Sauerstoffquelle zu dem Reaktor; und
(e) Wiederholen der Schritte (a) bis (d) in alternierender Weise.

18. Verfahren nach Anspruch 17, wobei das Atomverhältnis in dem Katalysator größer als 15/1 ist und bevorzugt größer als 50/1.

19. Verfahren nach Anspruch 17, wobei das Verfahren in gepulster Weise in einem Reaktorbett durchgeführt wird oder alternativ, wobei das Verfahren in einem Schwingbetrieb unter Verwendung mehrerer Reaktorbetten durchgeführt wird, oder alternativ mittels eines Reaktors mit einem zirkulierenden Katalysatorbett durchgeführt wird.

20. Verfahren nach Anspruch 17, wobei die Halogenquelle bis zu einem Umsatz von größer als 95 Molprozent umgesetzt wird.

21. Verfahren nach Anspruch 17, wobei die Sauerstoffquelle zu einer Umsetzung von größer als 95 Molprozent umgesetzt wird.

22. Verfahren nach Anspruch 17, wobei die Halogenquelle und die Sauerstoffquelle jeweils beide zu einem Umsatz von größer als 95 Molprozent umgesetzt werden.

23. Verfahren nach Anspruch 17, wobei ein Produktstrom, umfassend unumgesetzte C₁-Kohlenwasserstoff-Reaktanden, halogeniertes C₁-Produkt, Wasser und, wenn überhaupt, Restmengen an Halogenquelle und Sauerstoffquelle als Ausfluss aus dem Reaktor, erhalten wird; und von diesem Produktstrom halogeniertes C₁-Produkt und Wasser abgeschieden werden, was zu einem Recyclestrom, umfassend unumgesetzten C₁-Kohlenwasserstoff-Reaktanden und, wenn überhaupt, Restmengen an Halogenquelle und Sauerstoffquelle, führt; und der Recyclestrom direkt zum Reaktor in Schritt (c) recycliert wird.

24. Verfahren nach Anspruch 17, wobei der C₁-Kohlenwasserstoff-Reaktand Methan ist; die Halogenquelle Chlorwasserstoff ist; und der halogenierte C₁ Kohlenwasserstoff Methylchlorid ist; und wobei gegebenenfalls das Methylchlorid in einem nachgeschalteten Verfahren, um Methanol, ein leichtes Olefin, Benzin, Vinylchloridmonomer oder Essigsäure herzustellen, eingesetzt wird.

25. Verfahren nach Anspruch 17, wobei die Temperatur größer ist als 375 °C und kleiner als 700 °C.

26. Verfahren nach Anspruch 22, wobei der C₁-Kohlenwasserstoff-Reaktand Methan ist; die Halogenquelle Chlorwasserstoff ist; und der halogenierte C₁ Kohlenwasserstoff Methylchlorid ist.

## Revendications

1. Procédé d'halogénation oxydante, comportant le fait de mettre, dans un réacteur, un réactif de type hydrocarbure en C₁, choisi parmi le méthane, un hydrocarbure en C₁ halogéné et un mélange de tels composés, en contact avec une source d'halogène et une source d'oxygène, en présence d'un catalyseur, dans lequel procédé :
- le rapport molaire du réactif hydrocarbure en C₁ à la source d'halogène, dans un courant d'alimentation envoyé dans le réacteur, est supérieur à 23/1 ;
- ou le rapport molaire du réactif hydrocarbure en C₁ à la source d'oxygène, dans un courant d'alimentation envoyé dans le réacteur, est supérieur à 46/1 ;
- ou bien, dans les deux courants d'alimentation envoyés dans le réacteur, le rapport molaire du réactif hydrocarbure en C₁ à la source d'halogène est supérieur à 23/1 et le rapport molaire du réactif hydrocarbure en C₁ à la source d'oxygène est supérieur à 46/1 ;
- on effectue la mise en contact dans des conditions de procédé appropriées pour préparer un produit en C₁ halogéné qui comporte au moins un atome d'halogène de plus, en tant que substituant, par rapport à l'hydrocarbure employé comme réactif ;
- et le catalyseur comprend un halogénure de terre rare ou un oxyhalogénure de terre rare qui ne contient pratiquement ni fer ni cuivre, au point que le rapport en atomes de l'élément terre rare aux fer et cuivre est supérieur à 10/1, sous réserve que s'il y a du cérium dans le catalyseur, il y ait aussi au moins un autre élément terre rare dans le catalyseur.

2. Procédé conforme à la revendication 1, dans lequel, dans le catalyseur, ledit rapport en atomes est supérieur à 15/1, et de préférence supérieur à 50 /1,

3. Procédé conforme à la revendication 1, dans lequel le réactif de type hydrocarbure en C₁ est choisi dans l'ensemble constitué par les méthane, chlorométhane, bromométhane, iodométhane, dichlorométhane, dibromométhane, diiodométhane et chlorobromométhane, ainsi que les mélanges de ces composés.

4. Procédé conforme à la revendication 1, dans lequel la source d'halogène est choisie dans l'ensemble constitué par les halogènes sous forme élémentaire, les halogénures d'hydrogène, et les hydrocarbures halogénés qui portent un ou plusieurs atome(s) d'halogène labile(s) en tant que substituant(s).

5. Procédé conforme à la revendication 1, dans lequel la source d'halogène est du chlorure d'hydrogène.

6. Procédé conforme à la revendication 1, dans lequel on opère avec un rapport molaire du réactif hydrocarbure en C₁ à la source d'halogène supérieur à 30/1.

7. Procédé conforme à la revendication 1, dans lequel la source d'oxygène est choisie dans l'ensemble constitué par de l'oxygène moléculaire et de l'air, et l'on opère avec un rapport molaire du réactif hydrocarbure en C₁ à la source d'oxygène supérieur à 50/1.

8. Procédé conforme à la revendication 1, dans lequel il y a en plus un diluant, qui est choisi dans l'ensemble formé par de l'azote, de l'hélium, de l'argon, du monoxyde de carbone et du dioxyde de carbone, ainsi que les mélanges de ces corps.

9. Procédé conforme à la revendication 8, dans lequel on utilise le diluant en une quantité qui représente plus de 10 % et moins de 90 % du nombre total de moles du réactif hydrocarbure et du diluant.

10. Procédé conforme à la revendication 1, dans lequel l'halogénure de terre rare ou l'oxyhalogénure de terre rare est représenté par la formule suivante :
MO_{y}X_{z}
dans laquelle M représente un métal ou plusieurs métaux des terres rares, O représente l'oxygène, l'indice y est un nombre qui vaut de 0 à 1,5, X représente un ion halogénure, et l'indice z est un nombre qui vaut de plus de 0 à 3,0.

11. Procédé conforme à la revendication 10, dans lequel M représente le lanthane ou un mélange de lanthane et d'autres éléments des terres rares, et X représente l'ion chlorure.

12. Procédé conforme à la revendication 10, dans lequel l'halogénure de terre rare est représenté par la formule MX₃ et l'oxyhalogénure de terre rare est représenté par la formule MOX, dans lesquelles formules M représente au moins un élément des terres rares choisi dans l'ensemble constitué par les lanthane, cérium, néodyme, praséodyme, dysprosium, samarium, yttrium, gadolinium, erbium, ytterbium, holmium, terbium, europium, thulium et lutétium, ainsi que leurs mélanges, et X représente l'ion chlorure, bromure ou iodure.

13. Procédé conforme à la revendication 1, dans lequel on opère à une température supérieure à 375°C et inférieure à 700 °C.

14. Procédé conforme à la revendication 1, dans lequel le taux de conversion de la source d'halogène est supérieur à 95 % en moles.

15. Procédé conforme à la revendication 1, dans lequel le taux de conversion de la source d'oxygène est supérieur à 95 % en moles.

16. Procédé conforme à la revendication 1, dans lequel le réactif hydrocarbure en C₁ est du méthane, la source d'halogène est du chlorure d'hydrogène et le produit en C₁ halogéné est du chlorure de méthyle, lequel chlorure de méthyle sert éventuellement dans un procédé ultérieur de préparation de méthanol, d'une oléfine légère, d'une essence, de monomère chlorure de vinyle, ou d'acide acétique.

17. Procédé de production d'un produit en C₁ halogéné, lequel procédé comporte les étapes suivantes :
a) introduire un courant d'une source d'halogène dans un réacteur contenant un catalyseur, lequel catalyseur comprend un halogénure de terre rare ou un oxyhalogénure de terre rare, lequel halogénure ou oxyhalogénure de terre rare ne contient pratiquement ni fer ni cuivre, au point que le rapport en atomes de l'élément terre rare aux fer et cuivre est supérieur à 10/1, sous réserve que s'il y a du cérium dans le catalyseur, il y ait aussi au moins un autre élément terre rare dans le catalyseur ;
b) interrompre l'introduction du courant de source d'halogène dans le réacteur ;
c) introduire dans le réacteur un courant d'un mélange comprenant un réactif de type hydrocarbure en C₁, choisi parmi le méthane, un hydrocarbure en C₁ halogéné et un mélange de tels composés, et une source d'oxygène, de sorte que dans ce courant, la concentration de la source d'halogène est inférieure à 0,5 % en volume et le rapport molaire du réactif hydrocarbure en C₁ à la source d'halogène est supérieur à 23/1, et dans des conditions de procédé appropriées pour préparer un produit en C₁ halogéné qui comporte au moins un atome d'halogène de plus, en tant que substituant, par rapport à l'hydrocarbure employé comme réactif ;
d) interrompre l'introduction du courant de mélange comprenant le réactif hydrocarbure en C₁ et la source d'oxygène dans le réacteur ;
e) et répéter les étapes (a) à (d) en mode alterné.

18. Procédé conforme à la revendication 17, dans lequel, dans le catalyseur, ledit rapport en atomes est supérieur à 15/1, et de préférence supérieur à 50 /1.

19. Procédé conforme à la revendication 17, dans lequel on opère en mode pulsé dans un seul lit de réacteur, ou bien l'on opère en mode oscillant en utilisant plusieurs lits de réacteur ou en utilisant un réacteur à lit catalytique circulant.

20. Procédé conforme à la revendication 17, dans lequel on fait réagir la source d'halogène jusqu'à un taux de conversion supérieur à 95 % en moles.

21. Procédé conforme à la revendication 17, dans lequel on fait réagir la source d'oxygène jusqu'à un taux de conversion supérieur à 95 % en moles.

22. Procédé conforme à la revendication 17, dans lequel on fait réagir la source d'halogène et la source d'oxygène à tel point que le taux de conversion de chacune d'elles est supérieur à 95 % en moles.

23. Procédé conforme à la revendication 17, dans lequel on obtient en tant qu'effluent sortant du réacteur un courant de produit comprenant du réactif hydrocarbure en C₁ non transformé, du produit en C₁ halogéné, de l'eau, et d'éventuels résidus de la source d'halogène et de la source d'oxygène, duquel courant de produit l'on sépare l'eau et le produit en C₁ halogéné, ce qui donne un courant de recyclage comprenant le réactif hydrocarbure en C₁ non transformé et les éventuels résidus de la source d'halogène et de la source d'oxygène, et l'on renvoie ce courant de recyclage dans le réacteur, directement à l'étape (c).

24. Procédé conforme à la revendication 17, dans lequel le réactif hydrocarbure en C₁ est du méthane, la source d'halogène est du chlorure d'hydrogène et le produit hydrocarbure en C₁ halogéné est du chlorure de méthyle, lequel chlorure de méthyle sert éventuellement dans un procédé ultérieur de préparation de méthanol, d'une oléfine légère, d'une essence, de monomère chlorure de vinyle, ou d'acide acétique.

25. Procédé conforme à la revendication 17, dans lequel on opère à une température supérieure à 375 °C et inférieure à 700 °C.

26. Procédé conforme à la revendication 22, dans lequel le réactif hydrocarbure en C₁ est du méthane, la source d'halogène est du chlorure d'hydrogène et le produit hydrocarbure en C₁ halogéné est du chlorure de méthyle.
